# EUROPÄISCHE PATENTSCHRIFT

(11) **EP 0 284 095 B1**
(45) Veröffentlichungstag und Bekanntmachung des Hinweises auf die Patenterteilung: **07.07.1993**
(21) Anmeldenummer: 88104853.2
(22) Anmeldetag: 25.03.1988
(51) Int. Cl.: A61B 5/02

(54) **Verfahren und Vorrichtung zur automatischen nichtinvasiven Blutdruckmessung**
Method and apparatus for automatic non-invasive blood pressure measurement
Procédé et appareil pour mesurer automatiquement par voie non-sanglante la pression sanguine

(30) Priorität: 27.03.1987 CS 2135/87
(43) Veröffentlichungstag der Anmeldung: 28.09.1988
(73) Patentinhaber: TNO BIOMEDICAL INSTRUMENTATION, 1105 AZ AMSTERDAM (NL)
(72) Erfinder: Penaz, Jan, CSc, MUDr., Brno (CS)
(74) Vertreter: Kraag, F., Ir.

(56) Entgegenhaltungen:
- EP-A- 0 078 090
- US-A- 4 524 777
- IEEE TRANSACTIONS ON BIOLOGICAL ENGINEERING, Band BME-27, Nr. 3, März 1980, Seiten 150-155, New York, US; K. YAMAKOSHI et al.: "Indirect measurement of instantaneous arterial blood pressure in the human finger by the vascular unloading technique"

## Beschreibung

Die Erfindung betrifft ein Verfahren sowie eine Vorrichtung zur automatischen nichtinvasiven Blutdruckmessung, insbesondere beim Menschen, gemäß den Oberbegriffen der Ansprüche 1 bzw. 7, wie sie z.B. aus CS-A-133 205 bekannt sind.

Der aus CS-A-133 205 bekannte Blutdruckmesser umfaßt eine Druckmanschette oder eine Pelotte als Druckeinrichtung für die betreffende Arterie, die zugleich ein abgeschlossenes Meßvolumen vorgibt, einen plethysmographischen Sensor, der in der Druckeinrichtung vorgesehen ist und über wenigstens einen Verstärker und ggfs. einen Phasenkorrektor an einen elektrischen bzw. elektromechanischen Druckwandler angeschlossen ist, der den Druck in der Druckeinrichtung kontinuierlich und augenblicklich in einem geschlossenen Regelkreis so regelt, daß das Arterienvolumen auf einem Wert bleibt, der dem Nullwert der Arterienwandspannung entspricht. Der Druck in der Druckeinrichtung folgt dann fortlaufend dem Momentanwert des intraarteriellen Drucks.

Ähnliche Geräte sind sowohl aus der Patentliteratur (US-A-4 510 940) als auch aus Artikeln in medizinischen und technischen Zeitschriften bekannt (J. Pe 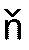 áz, Photoelectric measurement of blood pressure, volume and flow in the finger, 10^{th} Int. Conf. Med. Biol. Eng. Dresden, 1973, Seite 104). Die bekannten Geräte haben jedoch entweder keine automatische anfängliche Einstellung des Arbeitspunktes und der Verstärkung oder keine Korrektion dieser Parameter während der Messung. Es ist zwar schon eine Vorrichtung mit einer solchen Korrektion bekannt, die jedoch unter kurzer Unterbrechung der Messung durchgeführt werden muß. Ferner ist eine Vorrichtung mit einem zweiten, auf einer anderen, gefäßmäßig gleichwertigen Stelle lokalisierten Sensor bekannt, bei der folglich zwei Sensoren erforderlich sind. Die bekannten Geräte ermöglichen überdies nur die Blutdruckmessung an Fingerarterien bzw. an anderen leicht durchleuchtbaren Stellen und erlauben keine fortlaufende Messung an anderen, insbesondere größeren Arterien. Bei derartigen bekannten Vorrichtungen ist folglich eine automatische Einstellung bzw. Regelung des Arbeitspunktes und der Verstärkung auf einen optimalen Wert nur unter sehr einschränkenden Voraussetzungen möglich.

Der Erfindung liegt die Aufgabe zugrunde, die angeführten Nachteile zu beseitigen und ausgehend vom oben erläuterten Stand der Technik ein Verfahren sowie eine Vorrichtung zur automatischen nichtinvasiven Blutdruckmessung mit kontinuierlicher automatischer Einstellung und Regelung des Arbeitspunktes und ggfs. der Verstärkung anzugeben.

Die Aufgabe wird anspruchsgemäß gelöst. Die abhängigen Ansprüche betreffen vorteilhafte Ausführungsformen.

Das erfindungsgemäße Verfahren zur nichtinvasiven Messung des Blutdrucks in von der Oberfläche her komprimierbaren Arterien wird durchgeführt durch
- Komprimieren der Arterie in einem abgeschlossenen Meßvolumen,
- kontinuierliche plethysmographische Erfassung des Arterienvolumens im Meßvolumen
   und
- Konstanthaltung des Arterienvolumens auf einem Wert, bei dem die Arterienwandspannung gleich Null ist, durch kontinuierliche und augenblickliche Änderung des Drucks im Meßvolumen in Abhängigkeit vom erfaßten Arterienvolumen in einem geschlossenen Regelkreis unter gleichzeitiger Messung des Drucks im Meßvolumen;
   es ist dadurch gekennzeichnet, daß
- dem an das Meßvolumen angelegten Druck Druckschwingungen einer Frequenz überlagert werden, die höher ist als die Frequenz der höchsten harmonischen Komponente der Blutdruckpulswellen, aus den von diesen Druckschwingungen ausgelösten, mit einem Sensor erfaßten Gefäßvolumenoszillationen ein Korrektionssignal abgeleitet wird,
   und
- der Druck im Meßvolumen vorzugsweise in Abhängigkeit von Amplitude oder Phase der resultierenden Schwingungen des Arterienvolumens unter Berücksichtigung dieses Korrektionssignal kontinuierlich so korrigiert wird, daß die Arterienwandspannung gleich Null ist.

Der erfindungsgemäße automatische Blutdruckmesser zur nichtinvasiven Messung des Blutdrucks in von der Oberfläche her komprimierbaren Arterien, der insbesondere zur Durchführung des oben definierten Verfahrens vorgesehen ist, umfaßt
- eine plethysmorgraphische Druckeinrichtung, die ein Meßvolumen vorgibt,
- einen in der Druckeinrichtung im Meßvolumen vorgesehenen plethysmographischen Sensor,
- einen elektrischen Druckwandler, der durch das in mindestens einem Verstärker verstärkte sowie ggfs. einem Phasenkorrektor korrigierte Ausgangssignal des Sensors gesteuert und über eine Fluidleitung mit der Druckeinrichtung verbunden ist und einen dem Ausgangssignal des Sensors entsprechenden Druck erzeugt, wobei die Druckeinrichtung, der Sensor, der Verstärker und der Druckwandler einen geschlossenen Regelkreis bilden,
   sowie
- eine Einrichtung zur Einstellung bzw. Regelung des Arbeitspunkts und ggfs. der Verstärkung des Regelsystems;
   er ist gekennzeichnet durch
- einen Druckschwingungsgenerator, der direkt oder über den Druckwandler Druckschwingungen einer Frequenz erzeugt, die höher ist als die Frequenz der höchsten harmonischen Komponente der Blutdruckpulswellen, wobei der Druckschwingungsgenerator im Fall der direkten Erzeugung der Druckschwingungen über eine Fluidleitung mit der Druckeinrichtung verbunden ist,
   und
- eine Korrektionsschaltung, deren Eingang direkt oder über den im Regelkreis vorgesehenen Verstärker mit dem Sensor verbunden und deren Ausgang mit einem im Regelkreis zwischen dem Sensor und dem Verstärker vorgesehenen Summierglied verbunden sind und die ein Korrektionssignal erzeugt, mit dem das Ausgangssignal des Sensors über das Summierglied korrigiert wird.

Die Druckeinrichtung ist bevorzugt eine Druckmanschette oder eine Pelotte, die das Meßvolumen vorgibt. Der plethysmographische Sensor ist vorzugsweise ein Impedanzsensor und besonders bevorzugt ein photoelektrischer Reflexionssensor.

Nach einer bevorzugten Ausführungsform ist der Druckschwingungsgenerator als Oszillator ausgebildet, dessen Ausgang über ein zweites Summierglied mit dem Druckwandler verbunden ist, und die Korrektionsschaltung besteht aus einem Schmalbandverstärker, der auf die Frequenz des Oszillators abgestimmt ist, und einem Detektor, dessen Ausgang an eine Auswertungsschaltung angeschlossen ist, deren Ausgang über einen Integrator mit entsprechend vorgegebenen Vorzeichen am Summierglied anliegt.

Ein Verfahren und eine Vorrichtung gemäß den Oberbegriffen der unabhängigen Ansprüche 1 bzw. 7 sind aus IEEE Transactions on Biomedical Engineering, Band BME-27, Nr. 3, März 1980, Seiten 150-155 bekannt. Eine automatische kontinuierliche Korrektur des Arbeitspunkts des Servosystems zur Konstanthaltung des Arterienvolumens über die Ermittlung der Gefäßkomplianz während der Blutdruckmessung ist bei diesem Stand der Technik nicht vorgesehen.

Das Wesen der Erfindung liegt entsprechend darin, daß an den Grundregelkreis ein Druckschwingungsgenerator, der Druckschwingungen mit einer Frequenz erzeugt, die höher ist als die der höchsten harmonischen Komponente der Blutdruckpulswelle, und ferner eine Korrektionsschaltung angeschlossen sind, deren Eingang entweder direkt oder über wenigstens einen Verstärker an den plethysmographischen Sensor angeschlossen ist, wobei der Ausgang der Korrektionsschaltung an den Eingang der Schaltung zur Regelung des Arbeitspunktes des Grundregelkreises angeschlossen ist.

Erfindungsgemäß sind also, im Unterschied zu bekannten, gattungsgemäßen Vorrichtungen, ein Druckschwingungsgenerator, dessen Druckschwingungen dem Grundverlauf des Manschetten- oder Pelottendrucks überlagert sind, und eine Korrektionsschaltung vorgesehen, die fortlaufend die von diesen Druckschwingungen ausgelösten Gefäßvolumenoszillationen erfaßt und aus deren Amplitude oder Phase im Verlauf jedes Pulses ein Korrektionssignal ableitet. Das Korrektionssignal wird, vorzugsweise nach Integration, in den Grundregelkreis eingeführt, wodurch sein Arbeitspunkt, d.h. der Kompressionsgrad des gemessenen Gefäßes, kontinuierlich geregelt wird. Die Korrektionsschaltung mißt tatsächlich neben dem Blutdruck die sogenannte dynamische Gefäßkomplianz, die signifikant mit der Gefäßwandspannung zusammenhängt, und regelt diese auf einen Wert, der für die transmurale Druckübertragung optimal ist. Nach einem ähnlichen Prinzip, d.h. mit Messung der durch die Druckschwingungen erzeugten Gefäßvolumenoszillationen, arbeitet auch die Schaltung zur automatischen Regelung der Verstärkung gemäß der Erfindung. Besonders vorteilhaft ist der plethysmographische Sensor der Druckmanschette oder -pelotte, im Unterschied zu bekannten ähnlichen Geräten, als photoelektrischer Reflexionssensor ausgebildet.

Die erfindungsgemäße Vorrichtung besitzt im Vergleich mit den gattungsgemäßen herkömmlichen Blutdruckmessern eine Reihe von Vorteilen. Die Arbeitspunkt- und Verstärkungskorrektion ist kontinuierlich, d.h., die Blutdruckmessung verläuft ohne periodische Unterbrechungen. Die Korrektion wird vom Signal ein und desselben Sensors abgeleitet, der dem Grundregelkreis zugehört, so daß also der Einsatz eines weiteren, zweiten Sensors nicht erforderlich ist. Die erfindungsgemäße Vorrichtung ist somit einfacher aufgebaut und auch einfacher zu bedienen. Die Korrektion ist sehr wirksam und rasch, so daß bei der erfindungsgemäßen Vorrichtung kein Algorithmus für die Anfangseinstellung des Arbeitspunktes benötigt wird. Die Anwendung eines photoelektrischen Reflexionssensors für die Gefäßvolumenmessung ermöglicht ferner eine nichtinvasive Blutdruckmessung nicht nur an Fingerarterien, sondern auch an anderen von der Oberfläche zugänglichen Arterien, was einen besonderen Vorteil darstellt.

Die Erfindungskonzeption wird im folgenden anhand eines Ausführungsbeispiels unter Bezug auf die Zeichnungen näher erläutert, wobei Fig. 1 ein Blockschema der Grundanordnung, Fig. 2 ein Blockschema einer konkreten Ausführungsform und Fig. 3 ein Diagramm zur Erläuterung des erfindungsgemäßen Verfahrens bzw. der Funktion der erfindungsgemäßen Vorrichtung darstellen.

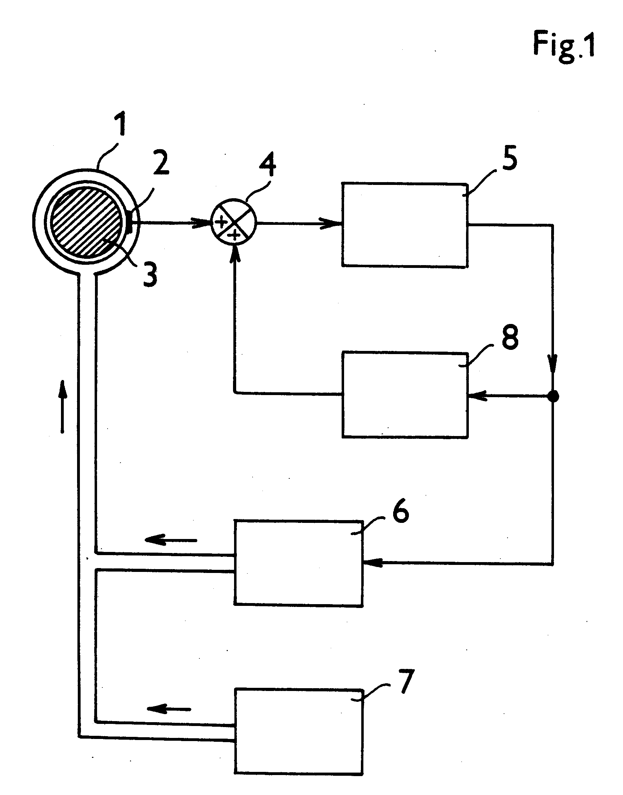

Die Grundanordnung ist in Fig. 1 dargestellt. Die mit dem plethysmographischen Sensor 2 des Arterienvolumens versehene, z. B. als Druckmanschette ausgebildete Druckeinrichtung 1 umgibt das Meßgebiet 3. Der plethysmographische Sensor 2 ist über ein Summierglied 4 an den Verstärker 5 angeschlossen, dessen Ausgang mit dem elektrischen bzw. elektromechanischen oder elektrohydraulischen Druckwandler 6 verbunden ist. Die Druckeinrichtung 1 ist über eine Fluidleitung einerseits mit dem Druckwandler 6 und andererseits mit einem Druckschwingungsgenerator 7 verbunden. An den Ausgang des Verstärkers 5 ist ferner eine Korrektionsschaltung 8 angeschlossen, deren Ausgang mit dem Summierglied 4 verbunden ist.

Die Druckeinrichtung 1, das Summierglied 4, der Verstärker 5 und der Druckwandler 6 bilden den Grundregelkreis, mit dem das Arterienvolumen im Meßgebiet 3 durch augenblickliche Druckänderungen in der Druckeinrichtung 1 auf einem konstanten Wert gehalten wird. Die Arterie ist dabei so komprimiert, daß die Arterienwand Nulltension hat. Nur unter dieser Bedingung entspricht der Druck in der Druckeinrichtung 1 dem intraarteriellen Druck. Dieser Zustand wird mittels der vom Druckschwingungsgenerator 7 erzeugten Druckschwingungen überwacht, wobei die Korrektionsschaltung 8 das erforderliche Korrektionssignal erzeugt. Dieses Korrektionssignal wird in entsprechender Polarität dem Summierglied zugeführt, so daß der Kompressionsgrad des Arterienvolumens kontinuierlich berichtigt und damit die Bedingung der Nulltension der Arterienwand erfüllt ist.

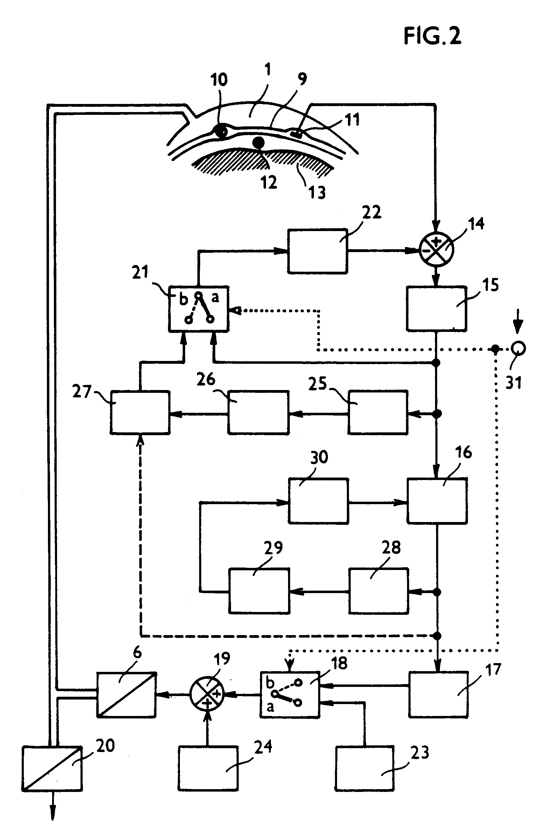

Das Blockschema von Fig. 2 zeigt ein konkretes Ausführungsbeispiel. Die als Druckmanschette ausgebildete Druckeinrichtung 1 ist auf einem geeigneten Gebiet angebracht, z.B. Finger, Handgelenk, Schläfengegend udgl., wo die Arterie 12 gegen eine natürliche Unterlage, z.B. den Knochen 13, oder eine künstliche feste Unterlage in weichem Gewebe gedrückt wird. Auf dem Innenblatt 9 der Druckmanschette sind eine Lichtquelle 10 und ein Photodetektor 11 so angeordnet, daß die Arterie 12 in ihrer Nähe bzw. zwischen ihnen liegt. Der Photodetektor 11 ist über ein erstes Summierglied 1 an den ersten Verstärker 16 angeschlossen, dessen Ausgang über einen ersten Schalter 21 und einen ersten Integrator 22 mit umgekehrter Polarität zum Summierglied 14 rückgeführt ist. Der Ausgang des ersten Verstärkers 15 ist außerdem an einen zweiten Verstärker 16 mit elektronischer Verstärkungsregelung angeschlossen, der über einen Phasenkorrektor 17 und einen zweiten Schalter 18 und ein zweites Summierglied 19 an den Druckwandler 6 angeschlossen ist, der mit der Druckeinrichtung 1 und einem Elektromanometer 20 verbunden ist. Der Ausgang des ersten Verstärkers 15 ist ferner an einen Schmalbandverstärker 25 angeschlossen, dessen Ausgang über einen ersten Detektor 26 mit einer Auswertungsschaltung 27 verbunden ist, die an den ersten Schalter 21 angeschlossen ist. Der Regeleingang der Auswertungsschaltung 27 ist an den Ausgang des zweiten Verstärkers 16 geschaltet. Dieser Ausgang ist ferner über einen Hochpaß 28 und einen zweiten Detektor 29 an einen zweiten Integrator 30 angeschlossen, dessen Ausgangsspannung die Verstärkung des zweiten Verstärkers 16 regelt. Der zweite Schalter 18 und der erste Schalter 21 werden mit einem externen Signal gesteuert, das am Regeleingang 31 zugeführt wird. Der zweite Schalter 18 ist mit einer Spannungsquelle 23, das zweite Summierglied 19 mit einem Oszillator 24 verbunden.

Die Funktion der Vorrichtung wird mittels des ersten Schalters 21 und des zweiten Schalters 18 gesteuert. Vor der eigentlichen Messung werden die Schalter 18, 21 in die Stellung a) geschaltet. In dieser Stellung der Schalter 18, 21 ist der Regelkreis des Grundservosystems aufgeschaltet, der Druck in der Druckeinrichtung 1 entspricht der Spannung der Spannungsquelle 23, und der Kreis der automatischen Nulleinstellung ist geschlossen, d.h., der erste Integrator 22 kompensiert nach und nach die Gleichstromkomponente der Ausgangsspannung des Photodetektors 11, die dem Arterienvolumen 12 entspricht. Nach einigen Sekunden wird die Ausgangsspannung des ersten Verstärkers 15 auf Null gesetzt, und der erste Schalter 21 und der zweite Schalter 18 werden in Stellung b) gebracht. Wenn das Korrektionssignal am Ausgang der Auswertungsschaltung 27 gleich Null ist, ändert sich die Ausgangsspannung des ersten Integrators 21 nicht, und das Servosystem hält das Ausgangssignal vom Photodetektor 11, das dem Volumen der Arterie 12 entspricht, auf dem ursprünglichen Wert. In dieser Situation löst jede Änderung des Arterienvolumens eine augenblickliche Druckänderung in der Druckeinrichtung 1 aus, welche die Volumenänderung der Arterie 12 kompensiert, und zwar bei geeigneter Verstärkung des zweiten Verstärkers 16 und bei Verwendung eines Phasenkorrektors 17 mit PID-Eigenschaften nahezu vollständig. Sollte der Anfangsdruck so gewählt worden sein, daß die Gefäßwandspannung gleich Null ist, entsprechen der Druck in der Druckeinrichtung 1 und somit auch die Ausgangsspannung des Elektromanometers 20 in jedem Moment dem intraarteriellen Druck.

In der Praxis kann man jedoch das adäquate anfängliche Gefäßvolumen, d.h. den Arbeitspunkt des Servosystems, nur mit Schwierigkeiten bestimmen; außerdem ist bekannt, daß sich sein Wert während der Messung ändern kann. Bei der erfindungsgemäßen Vorrichtung wird daher der Arbeitspunkt automatisch aufgesucht und ständig korrigiert, und zwar in der Weise, daß auf der erfaßten Druckkurve kleine Druckschwingungen überlagert werden, deren Frequenz höher ist als jede in der Blutdruckpulswelle natürlicherweise enthaltene Frequenz. Die Quelle dieser Druckschwingungen ist der Oszillator 24, dessen periodische Ausgangsspannung im zweiten Summierglied 19 zur Ausgangsspannung des Phasenkorrektors 17 addiert wird; der Druckwandler 6 erzeugt dann den benötigten Druckverlauf. Die überlagerten Druckschwingungen erzeugen dann in der Druckmanschette 1 winzige, aber meßbare Volumenoszillationen der Arterie 12, die zusammen mit dem ursprünglichen photoelektrischen Signal mit dem Photodetektor 11 registriert, dann im ersten Verstärker 15 verstärkt, danach abgetrennt und im Schmalbandverstärker 25 weiter verstärkt werden, der auf die Frequenz des Oszillators 24 abgestimmt ist; sie werden schließlich im ersten Detektor 26 erfaßt. Das Ausgangssignal dieses Detektors 26 entspricht dem physiologischen Parameter der dynamischen Gefäßkomplianz (DVC, dynamic vascular compliance), die direkt von der Arterienwandspannung abhängt.

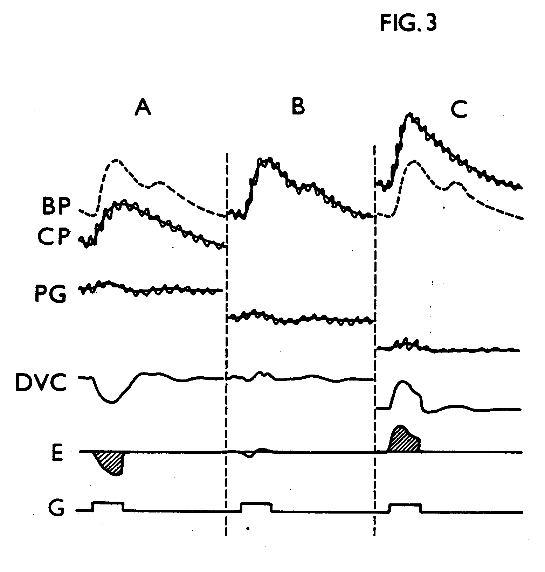

Wie Fig. 3 zeigt, wird die Restabweichung des Ausgangssignals des Photodetektors, d.h. die kleine Vergrößerung des Gefäßvolumens PG im systolischen Teil jeder Pulswelle von einer charakteristischen Änderung der DVC in Abhängigkeit von der Einstellung des Arbeitspunktes des Servosystems begleitet. Wie aus Teil A von Fig. 3 hervorgeht, ist, wenn der Arbeitspunkt unrichtig so gewählt wurde, daß das Gefäßvolumen PG durch das Servosystem auf einem zu hohen Wert gehalten wird, der registrierte Druck CP niedriger als der tatsächliche intraarterielle Druck BP (gestrichelte Kurve), und die DVC sinkt bei einer Vergrößerung des Gefäßvolumens entsprechend der Restregelabweichung. Der umgekehrte Fall ist in Teil C von Fig. 3 veranschaulicht. Wenn die Arterie durch das Servosystem zu stark komprimiert ist und das System einen unrichtig hohen Druck mißt, vergrößert sich die DVC am Anfang jedes Pulses merklich. Bei richtiger Einstellung des Blutdruckmessers gemäß Teil B von Fig. 3 hat das Gefäßvolumen PG einen mittleren Wert, und der registrierte Druck CP ist gleich dem intraarteriellen Druck BP. Die Auswertungsschaltung 27 von Fig. 2, die z.B. ein vom Signal der Regelrestabweichung geregelter getasteter Verstärker (gated amplifier) sein kann, das vom Ausgang des zweiten Verstärkers 16 gewonnen wird, registriert die Übergangsänderung der DVC am Anfang jedes Pulses, leitet von dieser Änderung das Fehlersignal E (Fig. 3) ab und führt es zum ersten Integrator 22, dessen Ausgangsspannung dann mittels des ersten Summierglieds 14 den Arbeitspunkt des Servosystems bestimmt. Damit wird der Grad der arteriellen Kompression automatisch und fortlaufend so korrigiert, daß die Gefäßwandspannung minimal ist. Die fortlaufende Verstärkung wird durch den zweiten Verstärker 16 (Fig. 2) mit elektronischer Regelung der Verstärkung sowie eine Schaltung gesichert, die aus dem Hochpaß 28, dem zweiten Detektor 29 und dem zweiten Integrator 30 besteht. Aus dem Ausgangssignal des zweiten Verstärkers 16 werden mit dem Hochpaß 28 die Frequenzen ausgewählt, die höher sind als die niedrigste vorausgesetzte Eigenfrequenz des Servosystems und auch die Frequenz der absichtlich eingeführten Druckschwingungen in der Druckeinrichtung. Diese Frequenzkomponenten werden verstärkt und mit dem zweiten Detektor 29 erfaßt und mit dem zweiten Integrator 30 integriert, dem auch eine konstante Spannung zugeführt wird, die den Sollwert der Verstärkung des Servosystems bestimmt. Die Ausgangsspannung des Integrators 30 hält die Verstärkung des zweiten Verstärkers 16 auf einem Wert, der mit Sicherheit niedriger ist als der Grenzwert, ab dem ungedämpfte Schwingungen des Servosystems auftreten würden.

## Patentansprüche

1. Verfahren zur nichtinvasiven Messung des Blutdrucks in von der Oberfläche her komprimierbaren Arterien
durch
- Komprimieren der Arterie in einem abgeschlossenen Meßvolumen,
- kontinuierliche plethysmographische Erfassung des Arterienvolumens im Meßvolumen
und
- Konstanthaltung des Arterienvolumens auf einem Wert, bei dem die Arterienwandspannung gleich Null ist, durch kontinuierliche und augenblickliche Änderung des Drucks im Meßvolumen in Abhängigkeit vom erfaßten Arterienvolumen in einem geschlossenen Regelkreis unter gleichzeitiger Messung des Drucks im Meßvolumen,
**dadurch gekennzeichnet,** daß
- dem an das Meßvolumen angelegten Druck Druckschwingungen einer Frequenz überlagert werden, die höher ist als die Frequenz der höchsten harmonischen Komponente der Blutdruckpulswellen,
aus den von diesen Druckschwingungen ausgelösten, mit einem Sensor erfaßten Gefäßvolumenoszillationen ein Korrektionssignal abgeleitet wird,
und
- der Druck im Meßvolumen unter Berücksichtigung dieses Korrektionssignals kontinuierlich so korrigiert wird, daß die Arterienwandspannung gleich Null ist.

2. Verfahren nach Anspruch 1, dadurch gekennzeichnet, daß die Verstärkung des dem erfaßten Arterienvolumen entsprechenden Meßsignals im geschlossenen Regelkreis automatisch geregelt wird.

3. Verfahren nach Anspruch 2, dadurch gekennzeichnet, daß der Druck im Meßvolumen bzw. die Verstärkung in Abhängigkeit von Amplitude oder Phase der Schwingungen des Arterienvolumens kontinuierlich geregelt wird.

4. Verfahren nach einem der Ansprüche 1 bis 3, dadurch gekennzeichnet, daß zur Komprimierung der Arterie eine Druckmanschette oder eine Pelotte verwendet werden, die das Meßvolumen vorgeben.

5. Verfahren nach einem der Ansprüche 1 bis 4, dadurch gekennzeichnet, daß das Arterienvolumen mit einem plethysmographischen Sensor auf der Basis eines Impedanzsensors oder eines photoelektrischen Reflexionssensors erfaßt wird.

6. Verfahren nach einem der Ansprüche 1 bis 5, dadurch gekennzeichnet, daß das der Amplitude oder Phase der Schwingungen des Arterienvolumens entsprechendes Korrektionssignal in den geschlossenen Regelkreis über ein darin nach dem Sensor vorgesehenes Summierglied eingeführt wird, dessen anderem Eingang das dem erfaßten Arterienvolumen entsprechende Meßsignal zugeführt wird.

7. Automatischer Blutdruckmesser zur nichtinvasiven Messung des Blutdrucks in von der Oberfläche her komprimierbaren Arterien zur Durchführung des Verfahrens nach einem der Ansprüche 1 bis 6, mit
- einer plethysmographischen Druckeinrichtung (1), die ein Meßvolumen vorgibt,
- einem in der Druckeinrichtung (1) im Meßvolumen vorgesehenen plethysmographischen Sensor (2),
- einem elektrischen Druckwandler (6), der durch das in mindestens einem Verstärker (5) verstärkte Ausgangssignal des Sensors (2) gesteuert und über eine Fluidleitung mit der Druckeinrichtung (1) verbunden ist und einen dem Ausgangssignal des Sensors (2) entsprechenden Druck erzeugt, wobei die Druckeinrichtung (1), der Sensor (2), der Verstärker (5) und der Druckwandler (6) einen geschlossenen Regelkreis bilden,
sowie
- einer Einrichtung zur Einstellung bzw. Regelung des Arbeitspunkts und ggfs. der Verstärkung des Regelsystems,
gekennzeichnet durch
- einen Druckschwingungsgenerator (7), der direkt oder über den Druckwandler (6) Druckschwingungen einer Frequenz erzeugt, die höher ist als die Frequenz der höchsten harmonischen Komponente der Blutdruckpulswellen, wobei der Druckschwingungsgenerator (7) im Fall der direkten Erzeugung der Druckschwingungen über eine Fluidleitung mit der Druckeinrichtung (1) verbunden ist,
und
- eine Korrektionsschaltung (8), deren Eingang direkt oder über den im Regelkreis vorgesehenen Verstärker (5) mit dem Sensor (2) verbunden und deren Ausgang mit einem im Regelkreis zwischen dem Sensor (2) und dem Verstärker (5) vorgesehenen Summierglied (4) verbunden sind und die ein Korrektionssignal erzeugt, mit dem das Ausgangssignal des Sensors (2) über das Summierglied (4) korrigiert wird.

8. Blutdruckmesser nach Anspruch 7, gekennzeichnet durch eine Korrektionsschaltung (8), die über ein entsprechendes Korrektionssignal den Druck im Meßvolumen bzw. die Verstärkung des Verstärkers in Abhängigkeit von Amplitude oder Phase der Schwingungen des Arterienvolumens so korrigiert, daß die Arterienwandspannung gleich Null ist.

9. Blutdruckmesser nach Anspruch 7 oder 8, dadurch gekennzeichnet, daß die Druckeinrichtung (1) eine Druckmanschette oder eine Pelotte ist, die das Meßvolumen vorgeben.

10. Blutdruckmesser nach einem der Ansprüche 7 bis 9, dadurch gekennzeichnet, daß der plethysmographische Sensor (2) ein Impedanzsensor oder ein photoelektrischer Reflexionssensor ist.

11. Blutdruckmesser nach einem der Ansprüche 7 bis 10, dadurch gekennzeichnet, daß
- der Druckschwingungsgenerator (7) als Oszillator (24) ausgebildet ist, dessen Ausgang über ein zweites Summierglied (19) mit dem Druckwandler (6) verbunden ist,
und
- die Korrektionsschaltung (8) aus einem Schmalbandverstärker (25), der auf die Frequenz des Oszillators (24) abgestimmt ist, und einem ersten Detektor (26) besteht, dessen Ausgang an eine Auswertungsschaltung (27) angeschlossen ist, die über einen ersten Integrator (22) vorzeichenrichtig an das Summierglied (14) angeschlossen ist.

12. Blutdruckmesser nach einem der Ansprüche 7 bis 11, dadurch gekennzeichnet, daß der Verstärker (5) des Grundregelkreises aus einem ersten Verstärker (15) und einem zweiten Verstärker (16) mit elektronischer Verstärkungsregelung besteht, der über einen Hochpaß (28) an einen zweiten Detektor (29) angeschlossen ist, dessen Ausgang über einen zweiten Integrator (30) mit dem Regeleingang für die Verstärkungsregelung des zweiten Verstärkers (16) verbunden ist.

13. Blutdruckmesser nach einem der Ansprüche 7 bis 12, dadurch gekennzeichnet, daß der plethysmographische Sensor (2) ein photoelektrischer Reflexionsplethysmograph mit einer Lichtquelle (10) und einem Photodetektor (11) ist, die im Innenblatt (9) einer Druckmanschette oder Pelotte als Druckeinrichtung (1) oder auf einer gegenüberliegenden festen Unterlage in der Nähe der gemessenen Arterie (12) angeordnet sind.

## Claims

1. A method for non-invasive measurement of the blood pressure in arteries being compressible from the outside, comprising the steps of
- compressing the artery in a sealed-off measurement volume,
- continuous plethysmographic detection of the arterial volume in the measurement volume, and
- maintaining the arterial volume at a value at which the tension of the arterial wall equals zero by continuously and instantaneously changing in a closed loop the pressure in the measurement volume in response to the detected arterial volume, with the pressure in the measurement volume being measured simultaneously,
**characterised in that**
- the pressure applied to the measurement volume is being superimposed by pressure oscillations having a frequency higher than the frequency of the highest harmonic component of the blood-pressure pulse waves,
- a correction signal is derived from oscillations of the arterial volume, which are caused by said pressure oscillations and detected by a sensor, and
- the pressure in the measurement volume is continuously corrected in response to said correction signal such that the tension of the arterial wall equals zero.

2. A method according to claim 1 characterised in that the amplification of the measurement signal corresponding to the detected arterial volume is automatically controlled in a closed loop.

3. A method according to claim 2 characterised in that the pressure in the measurement volume or the amplification is continuously controlled in relation to the amplitude or phase of the oscillations of the arterial volume.

4. A method according to anyone of claims 1 to 3 characterised in that a pressure sleeve or a truss pad defining the measurement volume are used for compressing the artery.

5. A method according to anyone of claims 1 to 4 characterised in that the arterial volume is detected by a plethysmographic sensor on the basis of an impedance sesor or a photoelectric reflexion sensor.

6. A method according to anyone of claims 1 to 5 characterised in that the correction signal corresponding to the amplitude or phase of the oscillations of the arterial volume is fed into the closed loop via an adder provided in the closed loop downstream of the sensor, the other input of the adder being supplied with the measurement signal corresponding to the detected arterial volume.

7. An automatic tonometer for non-invasive measurement of the blood pressure in arteries being compressible from the outside, to execute the process according to anyone of claims 1 to 6, comprising
- a plethysmographic pressure device (1) defining a measurement volume,
- a plethysmographic sensor (2) provided in the measurement volume defined by the pressure device (1),
- an electric pressure transformer (6) being controlled by the output signal of the sensor (2), which is amplified by at least one amplifier (5), and connected with the pressure device (1) via a fluid duct and producing a pressure corresponding to the output signal of the sensor (2), the pressure device (1), the sensor (2), the amplifier (5) and the pressure transformer (6) constituting a closed loop, and
- an arrangement for adjusting or controlling the operating point and, if required, amplifying the control system,
**characterised by**
- a pressure-oscillation generator (7) producing directly or via the pressure transformer (6) pressure oscillations having a frequency higher than the frequency of the highest harmonic component of the blood-pressure pulse waves, the pressure-oscillation generator (7) being connected with the pressure device (1) via a fluid duct in the case of direct production of the pressure oscillations, and
- a correction circuit (8) whose input is connected with the sensor (2) directly or via the amplifier (5) provided in the control loop and whose input is connected with an adder (4) provided in the control loop between the sensor (2) and the amplifier (5), the correction circuit (8) producing a correction signal used for correcting the output signal of the sensor (2) via the adder (4).

8. A tonometer according to claim 7 characterised by a correction circuit (8) which corrects the pressure in the measurement volume or the amplification of the amplifier in response to the amplitude or phase of the oscillations of the arterial volume by means of a corresponding correction signal such that the tension of the arterial wall equals zero.

9. A tonometer according to claim 7 or 8 characterised in that the pressure device (1) is a pressure sleeve or a truss pad defining the measurement volume.

10. A tonometer according to anyone of claims 7 to 9 characterised in that the plethysmographic sensor (2) is an impedance sensor or a photoelectric reflexion sensor.

11. A tonometer according to anyone of claims 7 to 10 characterised in that
- the pressure-oscillation generator (7) is formed as an oscillator (24) whose output is connected with the pressure transformer (6) via a second adder (19), and
- the correction circuit (8) comprises a narrow-band amplifier (25) tuned to the frequency of the oscillator (24) and a first detector (26) whose output is connected with an evaluation circuit (27) connected with the adder (14) via a first integrator (22) in a correctly signed manner.

12. A tonometer according to anyone of claims 7 to 11 characterised in that the amplifier (5) of the basic control circuit comprises a first amplifier (15) and a second amplifier (16) with electronic amplification control, connected via a high-pass filter (28) to a second detector (29) whose output is connected with the control input for controlling the amplification of the second amplifier (16) via a second integrator (30).

13. A tonometer according to anyone of claims 7 to 12 characterised in that the plethysmographic seonsor (2) is a photoelectric reflection plethysmograph comprising a light source (10) and a photo-sensitive cell (11) arranged within the inner layer (9) of a pressure sleeve or a truss pad serving as pressure device (1) or on the opposite side on a rigid support near the artery (12) to be measured.

## Revendications

1. Procédé pour mesurer par voie non-sanglante la pression sanguine dans les artères compressibles par leur surface, comprenant
- la compression de l'artère dans un volume de mesure renfermé,
- la détection pléthysmographique continue du volume d'artère dans le volume de mesure, et
- la maintenance du volume d'artère à une valeur constante qui implique que la tension de la paroi d'artère est de zéro, en changeant de façon continue et instantanée la pression dans le volume de mesure, en fonction du volume d'artére détecté, dans une boucle d'asservissement fermée, tout en mesurant simultanément la pression dans le volume de mesure,
caractérisé en ce qu'il y a superposition à la pression appliquée au volume de mesure des oscillations de pression ayant une fréquence supérieure à la fréquence de la composante harmonique la plus grande des ondes pulsatives de pression sanguine,
qu'un signal de correction est dérivé des oscillations du volume vasculaire causées par ces oscillations de pression et détectées par un détecteur, et
que la pression dans le volume de mesure est corrigée de façon continue, y entrant ce signal de correction, de la manière que la tension de la paroi d'artère soit de zéro.

2. Procédé selon la revendication 1,
caractérisé en ce que le gain du signal de mesure correspondant au volume d'artère détecté est réglée automatiquement dans la boucle d'asservissement fermée.

3. Procédé selon la revendication 2,
caractérisé en ce que la pression dans le volume de mesure ou le gain est réglée de façon continue en fonction de l'amplitude ou de la phase des oscillations du volume d'artère.

4. Procédé selon l'une des revendications 1 à 3,
caractérisé en ce qu'une manchette de compression ou une ceinture herniaire déterminant le volume de mesure sont utilisées pour la compression de l'artère.

5. Procédé selon l'une des revendications 1 à 4,
caractérisé en ce que le volume de l'artère est détecté au moyen d'un détecteur pléthysmographique fonctionnant à base d'un palpeur d'impédance ou d'un capteur photo-électrique à réflexion.

6. Procédé selon l'une des revendications 1 à 5,
caractérisé en ce que le signal de correction correspondant à l'amplitude ou la phase des oscillations du volume d'artère est introduit dans la boucle d'asservissement fermée par l'intermédiaire d'un élément d'addition prévu dans celle-ci en aval du détecteur, le signal de mesure correspondant au volume d'artère détecté étant amené à son autre entrée.

7. Tonomètre automatique pour mesurer par voie non-sanglante la pression sanguine dans les artères compressibles par leur surface, destiné à la réalisation du procédé selon l'une des revendications 1 à 6, comprenant
- un dispositif de compression pléthysmographique (1) déterminant un volume de mesure,
- un détecteur pléthysmographique (2) prévu dans le volume de mesure du dispositif de compression (1),
- un convertisseur de pression électrique (6) qui est commandé par le signal de sortie du détecteur (2) intensifié par au moins un amplificateur (5), et connecté, par l'intermédiaire d'une ligne fluidique, avec le dispositif de compression (1), et qui produit une pression correspondant au signal de sortie du détecteur (2), une boucle d'asservissement fermée étant formée par le dispositif de compression (1), le détecteur (2), l'amplificateur (5) et le convertisseur de pression (6), et
- un dispositif destiné à l'ajustage ou réglage du point de fonctionnement et, au besoin, du gain du système de réglage,
caractérisé par un générateur d'oscillations de pression (7) produisant, directement ou par l'intermédiaire d'un convertisseur de pression (6), des oscillations de pression ayant une fréquence supérieure à la fréquence de la composante harmonique la plus grande des ondes pulsatives de pression sanguine, le générateur d'oscillations de pression (7) étant connecté, dans le cas de génération directe des oscillations de pression, par l'intermédiaire d'une ligne fluidique avec le dispositif de compression (1), et
par un circuit de correction (8) dont l'entrée est connectée directement ou par l'intermédiaire de l'amplificateur (5) avec le détecteur (2) et dont la sortie est connectée avec un élément d'addition (4) prévu dans la boucle d'asservissement fermée entre le détecteur (2) et l'amplificateur (5), circuit qui produit un signal de correction servant à la correction du signal de sortie du détecteur (2) effectuée par l'intermédiaire de l'élément d'addition (4).

8. Tonomètre selon la revendication 7,
caractérisé par un circuit de correction (8) qui sert à corriger, par un signal de correction correspondant, la pression dans le volume de mesure ou le gain de l'amplificateur, en fonction de l'amplitude ou de la phase des oscillations du volume d'artère, de façon à ce que la tension de la paroi d'artère soit de zéro.

9. Tonomètre selon la revendication 7 ou 8,
caractérisé en ce que le dispositif de compression (1) se constitue d'une manchette de compression ou d'une ceinture herniaire déterminant le volume de mesure.

10. Tonomètre selon l'une des revendications 7 à 9,
caractérisé en ce que le détecteur pléthysmographique (2) est un palpeur d'impédance ou un capteur photo-électrique à réflexion.

11. Tonomètre selon l'une des revendications 7 à 10,
caractérisé en ce que le générateur d'oscillations de pression (7) est réalisé sous forme d'un oscillateur (24) dont la sortie est connectée, par l'intermédiaire d'un second élément d'addition (19), avec le convertisseur de pression (6), et
que le circuit de correction (8) se constitue d'un amplificateur à bande étroite (25) syntonisé en fréquence avec l'oscillateur (24) et d'un premier détecteur (26) dont la sortie est connectée avec un circuit d'analyse (27) relié avec signe de polarité correct, par l'intermédiaire d'un premier intégrateur (22), à l'élément d'addition (14).

12. Tonomètre selon l'une des revendications 7 à 11,
caractérisé en ce que l'amplificateur (5) de la boucle d'asservissement de base se constitue d'un premier amplificateur (15) und d'un second amplificateur (16) avec réglage électronique du gain relié, par l'intermédiaire d'un élément passe-haut (28), à un second détecteur (29) dont la sortie est connectée, par l'intermédiaire d'un second intégrateur (30), avec l'entrée de réglage destinée au réglage du gain du second amplificateur (16).

13. Tonomètre selon l'une des revendications 7 à 12,
caractérisé en ce que le détecteur pléthysmographique (2) est un pléthysmographe photo-électrique à réflexion avec une source lumineuse (10) et un photodétecteur (11) disposés dans la couche intérieure (9) d'une manchette de compression ou d'une ceinture herniaire constituant le dispositif de compression (1) ou en regard sur un appui fixe, voisin de l'artère (12) objet de la mesure.
